(19) Europäisches Patentamt
European Patent Office
Office européen des brevets

(11) EP 0 658 342 B1

(12) **EUROPEAN PATENT SPECIFICATION**

(45) Date of publication and mention of the grant of the patent: **17.03.1999 Bulletin 1999/11**

(51) Int. Cl.[6]: **A61K 31/335**, A61K 47/40, A61K 31/28

(21) Application number: **94114999.9**

(22) Date of filing: **23.09.1994**

(54) **Antineoplastic pharmaceutical composition containing a fumagillol derivative and a platinum complex**

Antineoplastische pharmazeutische Zusammensetzung enthaltend ein Fumagillolderivat und einen Platinkomplex

Composition pharmaceutique antineoplastique contenant des dérivées de fumagillol et un complex du platine

(84) Designated Contracting States:
**AT BE CH DE DK ES FR GB GR IE IT LI LU NL PT SE**

(30) Priority: **24.09.1993 JP 238341/93**

(43) Date of publication of application: **21.06.1995 Bulletin 1995/25**

(73) Proprietor:
**Takeda Chemical Industries, Ltd.**
**Osaka-shi, Osaka 541-0045 (JP)**

(72) Inventors:
- **Ikeyama, Shuichi**
  **Takatsuki, Osaka 569 (JP)**
- **Yamamoto, Toshihiro**
  **Suita, Osaka 565 (JP)**

(74) Representative:
**von Kreisler, Alek, Dipl.-Chem. et al**
**Patentanwälte,**
**von Kreisler-Selting-Werner,**
**Bahnhofsvorplatz 1 (Deichmannhaus)**
**50667 Köln (DE)**

(56) References cited:
**EP-A- 0 415 294** **EP-A- 0 461 427**
**EP-A- 0 470 569**

- **BIOCHEMICAL AND BIOPHYSICAL RESEARCH COMMUNICATIONS, vol.174, no.3, 1991 pages 1070 - 1076 MASAMI KUSAKA ET AL. 'Potent anti-angiogenic action of AGM-1470: comparison to the fumagillin parent'**
- **CANCER RESEARCH, vol.47, no.19, 1987 pages 5021 - 5024 KAN-EI LEE ET AL. 'Efficacy of antitumor chemotherapy in C3H mice enhanced by the antiangiogenesis steroid, cortisone acetate'**
- **WIEN. MED. WOCHENSCHR., vol.143, no.16, 1993 pages 454 - 457 M.MICKSCHE 'Neue Aspekte der Interferontherapie bei malignen Erkrankungen'**
- **CANCER RESEARCH, vol.52, 1992 BEVERLY A. TEICHER ET AL. 'Antiangiogenic agents potentiate cytotoxic cancer therapies against primary and metastatic disease'**
- **INT.J.CANCER, vol.57, no.6, 1994 pages 920 - 925 BEVERLY A. TEICHER ET AL. 'Potentiation of cytotoxic cancer therapies by TNP-470 alone and with other anti-angiogenic agents'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 658 342 B1

## Description

The present invention relates to an antineoplastic pharmaceutical composition for plural administrations. Specifically, it relates to an antineoplastic pharmaceutical composition for plural administrations which comprises a fumagillol derivative having angiogenesis inhibitory activity or a salt thereof (hereinafter sometimes referred to as TNP) in combination with a platinum complex having cytocidal activity.

### BACKGROUND OF THE INVENTION

Recent pharmacotherapies for cancer have mainly employed chemotherapeutic agents in order to kill tumor cells by their cytocidal activity. However, although presently available chemotherapeutic agents have useful pharmacological activities, they are also highly toxic to normal cells. Their anti-tumor activity is low at such a low dose that side effects are inhibited. Thus, the pharmacotherapies are unsatisfactory.

Recently, angiogenesis inhibitors have intensively been studied for novel pharmacotherapies of cancer, and many preclinical test results have been reported [e.g., Advances in Cancer Research, 43, 175 (1985)]. Angiogenesis is essential for the growth of solid tumor, and the growth of vascular endothelial cells is extremely vigorous in tumor tissues. On the other hand, in normal adults, angiogenesis is not observed except in tissues related to reproduction, and the turnover of vascular endothelial cells is very slow, i.e. on the order of months. Therefore, it is suggested that angiogenesis inhibitors which selectively inhibit the growth of vascular endothelial cells can be antineoplastic agents having new mechanisms of action and remarkably reduced side effects in comparison with conventional chemotherapeutic agents against cancer.

A fumagillin derivative, 6-O-(N-chloroacetylcarbamoyl)fumagillol, is an angiogenesis-specific inhibitor which has been found to exhibit cytostatic inhibitory effects on the growth of vascular endothelial cells in a wide range of low concentration (for example, 30pg/ml-3μg/ml) and selectively inhibit tube formation [Biochemical and Biophysical Research Communications, 174, 1070 (1991)]. Further, it has been reported that this compound exhibits tumor growth inhibitory activity and prolongs survival time based on the angiogenesis inhibitory activity when the compound is systemically administered to tumor-bearing animals having transplanted tumor cells against which the compound has low activity of direct tumor growth inhibition [Nature, 348, 555 (1990)].

Further, with regard to a combination of 6-O-(N-chloroacetylcarbamoyl)fumagillol and other drugs, therapeutic effects of a combination of the compound and the hormone preparation Tamoxifen on hormone-dependent breast carcinoma have been reported (International Journal of Oncology, 3, 525 (1993)). It would be desirable to provide an antineoplastic agent having more potent antineoplastic activity and higher safety than previously used agents or combinations thereof.

EP-A-415 294 describes fumagillol derivatives having angiogenesis inhibiting activity. Wien. Med. Wochenschr., vol. 143, no. 16-17, 1993, p. 454-457, Cancer Res., vol. 47, no. 19, 1987, p. 5021-5024 and Cancer Res., vol. 52, 1992, p. 6702-6704 describe combinations comprising antiangiogenic compounds with cisplatin.

### SUMMARY OF THE INVENTION

The present inventors have intensively studied to obtain an antineoplastic agent having more potent antineoplastic activity and higher safety than previously used agents or combinations thereof. As a result, it has been found that plural administrations of 6-O-(N-chloroacetylcarbamoyl)fumagillol in combination with cisplatin to cancer-bearing animals produce excellent therapeutic effects. After further studies based on this finding, the present invention has been completed.

The present invention provides

(1) an antineoplastic pharmaceutical composition for plural administrations which consists of a fumagillol derivative having angiogenesis inhibitory activity or a salt thereof (hereinafter sometimes referred to as "TNP") in combination with a platinum complex having cytocidal activity, and a pharmaceutically acceptable carrier or excipient;

(2) a kit of antineoplastic preparations for plural administration which comprises (1) a preparation comprising a fumagillol derivative having angiogenesis inhibitory activity or a salt thereof and a pharmaceutically acceptable carrier or excipient, and (2) a preparation comprising a platinum complex having cytocidal activity and a pharmaceutical carrier or excipient;

(3) the use of a composition consisting of a fumagillol derivative having angiogenesis inhibitory activity or a salt thereof in combination with a platinum complex having cytocidal activity for preparing a medicament for treating a tumor in a mammal;

(4) the use of a fumagillol derivative having angiogenesis inhibitory activity in combination with a platinum complex having cytocidal activity for formulation of an antineoplastic pharmaceutical composition which consists of a fumagillol derivative having angiogenesis inhibitory activity or a salt thereof in combination with a platinum complex having cytocidal activity, and if necessary has a pharmaceutically acceptable carrier or excipient for plural administration.

In a preferred embodiment of the present invention, the pharmaceutical composition as defined in (1) above is for treating tumor in a mammal and is suitable to be administered to such a mammal at least twice.

As used herein, "plural administrations" means that each of the fumagillol derivatives or a salt thereof having angiogenesis inhibitory activity and the platinum complex having cytocidal activity is administered to a subject at least twice.

These objects as well as other objects and advantages of the present invention will become apparent to those skilled in the art from the following description.

DETAILED DESCRIPTION OF THE INVENTION

In the present invention, any fumagillol derivatives having angiogenesis inhibitory activity can be used as long as they have selective cytostatic inhibitory activity against the growth of vascular endothelial cells.

Examples of the fumagillol derivative include a fumagillol derivative of the formula (I) (hereinafter sometimes referred to as the compound (I)):

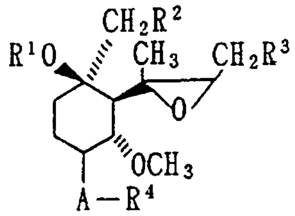

wherein $R^1$ is hydrogen; $R^2$ is halogen, $N(O)_mR^5R^6$, $N^+R^5R^6R^7 \cdot X^-$, $S(O)_nR^5$ or $S^+R^5R^6 \cdot X^-$, in which $R^5$, $R^6$ and $R^7$ represent independently an optionally substituted hydrocarbon group or an optionally substituted heterocyclic group; $X^-$ represents a counter anion; m represents 0 or 1; n represents an integer of 0 to 2; or $R^5$ and $R^6$ together with the adjacent nitrogen or sulfur atom may form an optionally substituted nitrogen- or sulfur-containing heterocyclic group which may form a condensed ring; or $R^1$ and $R^2$ are combined to represent a chemical bond; $R^3$ is an optionally substituted 2-methyl-1-propenyl group or an optionally substituted isobutyl group; A is oxygen or $NR^8$, in which $R^8$ represents hydrogen, an optionally substituted lower alkyl group or an optionally substituted aryl group; and $R^4$ is hydrogen, an optionally substituted hydrocarbon group or an optionally substituted acyl group; or a salt thereof.

In the above formula (I), halogen represented by $R^2$ includes fluorine, chlorine, bromine and iodine.

When $R^1$ and $R^2$ are combined to represent a chemical bond, an epoxy ring is formed.

The hydrocarbon group of the optionally substituted hydrocarbon group represented by $R^5$, $R^6$ or $R^7$ includes straight-chain or branched $C_{1-6}$ alkyl (e.g., methyl, ethyl, propyl, isopropyl, butyl, isobutyl, sec-butyl, pentyl, isopentyl, hexyl ), straight-chain or branched $C_{2-6}$ alkenyl (e.g., vinyl, allyl, 2-butenyl, methylallyl, 3-butenyl, 2-pentenyl, 4-pentenyl, 5-hexenyl ), straight-chain or branched $C_{2-6}$ alkynyl (e.g., ethynyl, propargyl, 2-butyn-1-yl, 3-butyn-2-yl, 1-pentyn-3-yl, 3-pentyn-1-yl, 4-pentyn-2-yl, 3-hexyn-1-yl ), $C_{3-6}$ cycloalkyl (e.g., cyclopropyl, cyclobutyl, cyclopentyl, cyclohexyl ), $C_{3-6}$ cycloalkenyl (e.g., cyclobutenyl, cyclopentenyl, cyclohexenyl, cyclohexadienyl ), $C_{7-13}$ aralkyl (e.g., benzyl, 1-phenethyl, 2-phenethyl ), and $C_{6-10}$ aryl (e.g., phenyl, naphthyl).

The heterocyclic group of the optionally substituted heterocyclic group represented by $R^5$, $R^6$ or $R^7$ includes 5- or 6-membered heterocyclic groups containing 1 to 4 heteroatoms (e.g., nitrogen, oxygen, sulfur), for example, furyl (e.g., 2-furyl ), thienyl (e.g., 2-thienyl ), thiazolyl (e.g., 4-thiazolyl ), imidazolyl (e.g., 4-imidazolyl ), pyridyl (e.g., 4-pyridyl ), thiadiazolyl (e.g., 1,3,4-thiadiazol-2-yl) and tetrazolyl (e.g., 5-tetrazolyl). Further, the heterocyclic group may be condensed with a 5- or 6-membered ring which may contain 1 to 3 heteroatoms (e.g., nitrogen, oxygen, sulfur ) other than carbon atoms (e.g., benzene, pyridine, cyclohexane ) to form a condensed bicyclic group (e.g., quinolyl (e.g., 8-quinolyl) or purinyl (e.g., 8-purinyl)).

The nitrogen-containing heterocyclic group which may be formed by $R^5$ and $R^6$ together with the adjacent nitrogen atom includes 4- to 7-membered nitrogen-containing heterocyclic groups which may contain 1 to 3 heteroatoms (e.g., nitrogen, oxygen, sulfur ) other than a nitrogen atom (e.g., pyrrolidinyl (e.g., pyrrolidin-1-yl ), piperidino, morpholino, piperazinyl (e.g., piperazin-1-yl)).

The sulfur-containing heterocyclic group which may be formed by $R^5$ and $R^6$ together with the adjacent sulfur atom includes 4- to 7-membered sulfur-containing heterocyclic groups which may contain 1 to 3 heteroatoms (e.g.,

nitrogen, oxygen, sulfur ) other than a sulfur atom (e.g., tetrahydrothiophenyl (e.g., tetrahydrothiophen-1-yl ), thioxanyl (e.g., 1,4-thioxan-1-yl)).

The nitrogen- or sulfur-containing heterocyclic group which may be formed by $R^5$ and $R^6$ together with the adjacent nitrogen or sulfur atom may be condensed with a 5-or 6-membered ring (e.g., benzene, pyridine, pyrazine, pyrimidine, pyridazine, cyclohexane ) to form a condensed bicyclic group (e.g., isoindolinyl (e.g., isoindolin-2-yl),

isoquinolyl (e.g., 2-isoquinolyl ), dihydrobenzo[c]thiophenyl (e.g.,1,3-dihydrobenzo[c]thiophen-2-yl, 2,3-dihydrobenzo[b]thiophen-1-yl ), tetrahydrobenzo[d]thiepinyl (e.g., 1,2,4,5-tetrahydrobenzo[d]thiepin-3-yl ), dihydrothieno[3,4-c]pyridinyl (e.g., 1,3-dihydrothieno[3,4-c]pyridin-2-yl),

dihydrothieno[3,4-b]pyrazinyl (e.g., 5,7-dihydrothieno[3,4-b]pyrazin-6-yl ), dihydrothieno[3,4-d]pyridazinyl (e.g., 5,7-dihydrothieno[3,4-d]pyridazin-6-yl)).

The lower alkyl group of the optionally substituted lower alkyl group represented by $R^8$ includes straight-chain or branched $C_{1-6}$ alkyl groups (e.g., methyl, ethyl, propyl, isopropyl, butyl, isobutyl, sec-butyl, pentyl, isopentyl, hexyl).

The aryl group of the optionally substituted aryl group represented by $R^8$ includes $C_{6-10}$ aryl groups (e.g., phenyl, naphthyl ).

The hydrocarbon group of the optionally substituted hydrocarbon group represented by $R^4$ includes that described above with respect to that of the optionally substituted hydrocarbon represented by $R^5$, $R^6$ or $R^7$.

When the hydrocarbon group represented by $R^4$ is an alkenyl group, it preferably has no substituent.

The optionally substituted acyl group represented by $R^4$ includes residues of optionally substituted acids, for example, acyl groups derived from the corresponding acids, such as carboxylic acid acyl, sulfonic acid acyl, carbamoyl, thiocarbamoyl and sulfamoyl each of which may have at least one substituent. Examples of the optionally substituted acyl include alkanoyl, aroyl, heterocyclic carbonyl, carbamoyl, thiocarbamoyl, arylsulfonyl, alkylsulfonyl, sulfamoyl, alkoxycarbonyl and aryloxycarbonyl each of which may have at least one substituent.

The alkanoyl group of the above optionally substituted alkanoyl group includes $C_{1-6}$ alkanoyl groups (e.g., formyl, acetyl, propionyl, isopropionyl, butyryl, pentanoyl, hexanoyl).

The aroyl group of the optionally substituted aroyl group includes $C_{7-11}$ aroyl groups (e.g., benzoyl, 1-naphthoyl, 2-naphthoyl).

The heterocyclic carbonyl group of the optionally substituted heterocyclic carbonyl group includes 5- or 6-membered heterocyclic carbonyl groups containing 1 to 4 heteroatoms (e.g., nitrogen, oxygen, sulfur), for example, furoyl (e.g., 2-furoyl), thenoyl (e.g., 2-thenoyl), nicotinyl and isonicotinyl .

The arylsulfonyl group of the optionally substituted arylsulfonyl group includes $C_{6-10}$ arylsulfonyl groups (e.g., benzenesulfonyl, 1-naphthylsulfonyl, 2-naphthylsulfonyl).

The alkylsulfonyl group of the optionally substituted alkylsulfonyl group includes $C_{1-6}$ alkylsulfonyl groups (e.g., methylsulfonyl, ethylsulfonyl).

The alkoxycarbonyl group of the optionally substituted alkoxycarbonyl group includes $C_{2-7}$ alkoxycarbonyl groups (e.g., methoxycarbonyl, ethoxycarbonyl, isobutoxycarbonyl).

The aryloxycarbonyl group of the optionally substituted aryloxycarbonyl group includes $C_{7-11}$ aryloxycarbonyl groups (e.g., phenoxycarbonyl, naphthyloxycarbonyl (e.g., 1-naphthyloxycarbonyl, 2-naphthyloxycarbonyl )).

Examples of the substituent of the optionally substituted 2-methyl-1-propenyl group or the optionally substituted isobutyl group represented by $R^3$ include hydroxyl, amino, lower ($C_{1-3}$) alkylamino (e.g., methylamino, ethylamino, isopropylamino ) and di-lower ($C_{1-3}$) alkylamino (e.g., dimethylamino, diethylamino). Hydroxyl and di-lower ($C_{1-3}$) alkylamino, particularly dimethylamino, are preferred.

The optionally substituted hydrocarbon group or optionally substituted heterocyclic group represented by $R^5$, $R^6$ or $R^7$; the optionally substituted nitrogen- or sulfur-containing heterocyclic group formed by $R^5$ and $R^6$ together with the adjacent nitrogen or sulfur atom which may be condensed with a further ring; the optionally substituted lower alkyl group or optionally substituted aryl group represented by $R^8$; as well as the optionally substituted hydrocarbon group and optionally substituted acyl group (e.g., alkanoyl, aroyl, heterocyclic carbonyl, carbamoyl, thiocarbamoyl, arylsulfonyl, alkylsulfonyl, sulfamoyl, alkoxycarbonyl, aryloxycarbonyl) represented by $R^4$ contain preferably 1 to 3 substituents.

Examples of such substituents include $C_{1-6}$ alkyl groups (e.g., methyl, ethyl, propyl, isopropyl, butyl, isobutyl, sec-butyl, pentyl, isopentyl, hexyl), $C_{2-6}$ alkenyl groups (e.g., vinyl, allyl, 2-butenyl, methylallyl, 3-butenyl, 2-pentenyl, 4-pentenyl, 5-hexenyl ), $C_{2-6}$ alkynyl groups (e.g., ethynyl, propargyl, 2-butyn-1-yl, 3-butyn-2-yl, 1-pentyn-3-yl, 3-pentyn-1-yl, 4-pentyn-2-yl, 3-hexyn-1-yl), $C_{3-6}$ cycloalkyl groups (e.g., cyclopropyl, cyclobutyl, cyclopentyl, cyclohexyl), $C_{3-6}$ cycloalkenyl groups (e.g., cyclobutenyl, cyclopentenyl, cyclohexenyl, cyclohexadienyl), $C_{6-10}$ aryl groups (e.g., phenyl, naphthyl), amino, mono-$C_{1-6}$ alkylamino groups (e.g., methylamino, ethylamino, isopropylamino), di-$C_{1-6}$ alkylamino groups (e.g., dimethylamino, diethylamino), azido, nitro, halogen (e.g., fluorine, chlorine, bromine, iodine), hydroxyl, $C_{1-4}$ alkoxy groups (e.g., methoxy, ethoxy), $C_{6-10}$ aryloxy groups (e.g., phenoxy, naphthyloxy), $C_{1-6}$ alkylthio groups (e.g., methylthio, ethylthio, propylthio ), $C_{6-10}$ arylthio groups (e.g., phenylthio, naphthylthio), cyano, carbamoyl, carboxyl, $C_{1-4}$ alkoxycarbonyl groups (e.g., methoxycarbonyl, ethoxycarbonyl), $C_{7-11}$ aryloxycarbonyl groups (e.g., phenoxycarbonyl, 1-naphthyloxycarbonyl, 2-naphthyloxycarbonyl),

carboxy-$C_{1-4}$ alkoxy groups (e.g., carboxymethoxy, 2-carboxyethoxy), $C_{1-6}$ alkanoyl groups (e.g., formyl, acetyl, propionyl, isopropionyl, butyryl, pentanoyl, hexanoyl),

$C_{7-11}$ aroyl groups (e.g., benzoyl, 1-naphthoyl, 2-naphthoyl), $C_{1-6}$ alkylsulfonyl groups (e.g., methylsulfonyl, ethylsulfonyl), $C_{6-10}$ arylsulfonyl groups (e.g., benzenesulfonyl, 1-naphthylsulfonyl, 2-naphthylsulfonyl), $C_{1-6}$ alkylsulfinyl groups (e.g., methylsulfinyl, ethylsulfinyl), $C_{6-10}$ arylsulfinyl groups (e.g., benzenesulfinyl, 1-naphthylsulfinyl, 2-naphthylsulfinyl), 5- or 6-membered heterocyclic groups containing 1 to 4 heteroatoms (e.g., nitrogen, oxygen, sulfur)

(e.g., furyl (e.g., 2-furyl), thienyl (e.g, 2-thienyl), thiazolyl (e.g., 4-thiazolyl), imidazolyl (e.g., 4-imidazolyl ), pyridyl (e.g., 4-pyridyl), thiadiazolyl (e.g., 1,3,4-thiadiazol-2-yl),

tetrazolyl (e.g., 5-tetrazolyl)), 5- or 6-membered heterocyclic carbonyl groups containing 1 to 4 heteroatom (e.g., nitrogen, oxygen, sulfur ) (e.g., furoyl (e.g., 2-furoyl), thenoyl (e.g., 2-thenoyl),

nicotinoyl, isonicotinoyl )), 5- or 6-membered heterocyclic thio groups containing 1 to 4 heteroatoms (e.g., nitrogen, oxygen, sulfur ) (e.g., pyridylthio (e.g., 4-pyridylthio, pyrimidylthio (e.g., 2-pyrimidylthio), thiadiazolylthio (e.g., 1,3,4-thiadiazol-2-ylthio ), tetrazolylthio (e.g., 5-tetrazolylthio )).

Further, the heterocyclic thio group may be condensed with a benzene ring to form a condensed bicyclic thio group (e.g., benzothiazolylthio (e.g., 2-benzothiazolylthio), quinolylthio (e.g., 8-quinolylthio)).
Furthermore, when $R^4$ represents a disubstituted carbamoyl, thiocabamoyl or sulfamoyl group, the substituents together with the nitrogen atom of the carbamoyl, thiocarbamoyl or sulfamoyl group may form a nitrogen-containing heterocyclic group (e.g., 4- to 7-membered nitrogen-containing heterocyclic groups which may contain 1 to 3 heteroatoms (e.g., nitrogen, oxygen, sulfur ) other than a nitrogen atom, such as pyrrolidinyl (e.g., pyrrolidin-1-yl), piperidino, morpholino, piperazinyl (e.g., piperazin-1-yl), methylpiperazinyl (e.g., 4-methylpiperazin-1-yl) and phenylpiperazinyl (e.g., 4-phenylpiperazin-1-yl)).

The substituent in the optionally substituted hydrocarbon group or optionally substituted heterocyclic group represented by $R^5$, $R^6$ or $R^7$; the substituent in the optionally substituted nitrogen- or sulfur-containing heterocyclic group which may be formed by $R^5$ and $R^6$ together with the adjacent nitrogen or sulfur atom and may be condensed with a further ring; the substituent in the optionally substituted lower alkyl group or optionally substituted aryl group represented by $R^8$; as well as the substituent in the optionally substituted hydrocarbon group or optionally substituted acyl group (e.g., alkanoyl, aroyl, heterocyclic carbonyl, carbamoyl, thiocarbamoyl, arylsulfonyl, alkylsulfonyl, sulfamoyl, alkoxycarbonyl, aryloxycarbonyl) represented by $R^4$ may further contain 1 to 3 substituents at the possible positions.

Examples of such substituents include the aforementioned $C_{1-6}$ alkyl groups, $C_{2-6}$ alkenyl groups, $C_{2-6}$ alkynyl groups, $C_{3-6}$ cycloalkyl groups, $C_{3-6}$ cycloalkenyl groups, $C_{6-10}$ aryl groups, amino, mono-$C_{1-6}$ alkylamino groups, di-$C_{1-6}$ alkylamino groups, azido, nitro, halogen, hydroxyl, $C_{1-4}$ alkoxy groups, $C_{6-10}$ aryloxy groups, $C_{1-6}$ alkylthio groups, $C_{6-10}$ arylthio groups, cyano, carbamoyl, carboxyl, $C_{1-4}$ alkoxycarbonyl groups, $C_{7-11}$ aryloxycarbonyl groups, carboxy-$C_{1-4}$ alkoxy groups, $C_{1-6}$ alkanoyl groups, halogeno $C_{1-6}$ alkanoyl groups, $C_{7-11}$ aroyl group, $C_{1-6}$ alkylsulfonyl groups, $C_{6-10}$ arylsulfonyl groups, $C_{1-6}$ alkylsulfinyl, $C_{6-10}$ arylsulfinyl groups, 5- or 6-membered heterocyclic groups, 5- or 6-membered heterocyclic carbonyl groups and 5- or 6-membered heterocyclic thio groups.

The counter anion represented by $X^-$ includes, for example, halogenide ions (e.g., iodide ion, bromide ion, chloride ion), sulfate ion, phosphate ion, nitrate ion, perchlorate ion, tetrafluoroborate ion, methanesulfate ion, p-tolylsulfate ion, benzenesulfate ion, hydroxyl ion and organic carboxylate ions (e.g., oxalate ion, maleate ion, fumarate ion, succinate ion, citrate ion, lactate ion, trifluoroacetate ion, lactobionate ion, acetate ion, propionate ion, tartrate ion, ethyl succinate ion ).

The compound (I) has asymmetric centers in its molecule and is optically active. Its absolute configuration is based on the starting material, fumagillol. The absolute configuration of the compound (I) is the same as that of fumagillol unless otherwise indicated. The mode of bonding of the substituents on the cyclohexane ring is as follows:

....., 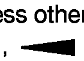 and ______ represent α-bond, β-bond and either α- or β-bond, respectively.

In the compound (I), preferably, $R^1$ and $R^2$ are combined to represent a chemical bond, or $R^1$ is hydrogen and $R^2$ is $N(O)_mR^5R^6$, $N^+R^5R^6R^7 \cdot X^-$, $S(O)_nR^5$ or $S^+R^5R^6 \cdot X^-$. More preferably, $R^2$ is $S^+R^5R^6 \cdot X^-$ in which $R^5$ and $R^6$ are independently a hydrocarbon group and $X^-$ is a halogenide ion. The compounds of the formula (I) wherein $R^1$ and $R^2$ are combined to represent a chemical bond are particularly preferred.

A is preferably oxygen or NH, more preferably oxygen.

$R^3$ is preferably a 2-methyl-1-propenyl or isobutyl group which is optionally substituted with (1) hydroxyl or (2) dialkylamino group. $R^3$ is more preferably 2-methyl-1-propenyl.

$R^4$ is preferably hydrogen or an optionally substituted carbamoyl. In particular, it is preferred when the carbamoyl is substituted with (1) a $C_{1-6}$ alkyl group or (2) a $C_{1-6}$ alkanoyl group optionally substituted with halogen.

Preferred examples of the compound (I) include 6-O-(N-chloroacetylcarbamoyl)fumagillol, 6α-(N'-chloroacetylureido)-6-deoxyfumagillol, 4-(N'-chloroacetylureido)-2-(1,2-epoxy-1,5-dimethyl-4-hexenyl)-1-(1,3-dihydrobenzo[c]thiophen-2-ylio)methyl-3-methoxycyclohexanol chloride and 6-O-(N-methylcarbamoyl)fumagillol. In particular, 6-O-(N-chloroacetylcarbamoyl)fumagillol and 6-O-(N-methylcarbamoyl)fumagillol are preferred.

When the compound (I) has an acidic substituent (e.g., carboxyl) or a basic substituent (e.g., amino, mono-lower alkylamino, di-lower alkylamino, nitrogen-containing heterocyclic group) in the molecule, the compound (I) may form a physiologically acceptable salt thereof. Examples of the physiologically acceptable salt include those with inorganic bases, organic bases, inorganic acids, organic acids, basic or acidic amino acids and the like. As the inorganic base which can form these salts, there are, for example, alkali metals (e.g., sodium, potassium) and alkaline earth metals (e.g., calcium, magnesium);

as the organic base, there are, for example, trimethylamino, triethylamine, pyridine, picoline, N,N-dibenzylethylenediamine, ethanolamine, diethanolamine, tris(hydroxymethyl)aminomethane and dicyclohexylamine;

as the inorganic acid, there are, for example, hydrochloric acid, hydrobromic acid, sulfuric acid, nitric acid and phosphoric acid; as the organic acid, there are, for example, formic acid, acetic acid, trifluoroacetic acid, oxalic acid, tartaric acid, fumaric acid, maleic acid, methanesulfonic acid, benzenesulfonic acid and p-toluenesulfonic acid; and as the basic or acidic amino acid, there are, for example, arginine, lysine, ornithine, aspartic acid and glutamic acid.

The above salts with bases (i.e., salts with inorganic bases, salts with organic bases, salts with basic amino acids) are formed by the bases and the carboxyl group in the substituent of the compound (I). The above salts with acids (i.e., salts with inorganic acids, salts with organic acids, salts with acidic amino acids) are formed by the acids and amino, mono-lower alkylamino groups, di-lower alkylamino groups or nitrogen-containing heterocyclic groups in the substituent of the compound (I).

When the compound (I) has a di-lower alkyl amino group, a nitrogen-containing heterocyclic group or a nitrogen-containing aromatic heterocyclic group, the nitrogen atom in these groups may be further alkylated to form a quaternary ammonio group (e.g., trimethylammonio, N-methylpyridinyl, N-methylpyrrolidin-1-ylium), and the counter anion thereof includes the counter anions shown with respect to those represented by $X^-$.

The compound represented by the formula (I) or a salt thereof can be produced by using, as a starting material, fumagillol [Tarbell, D. S. et al., J. Am. Chem. Soc., 83, 3096 (1961)] which is a hydrolyzate of fumagillin produced by a microorganism, by the processes described in, for example, EP-A-325,199, EP-A-359,036, EP-A-357,061, EP-A-386,667, EP-A-387,650, EP-A-415,294 and EP-A-354,787 or modifications thereof.

The fumagillol derivatives having angiogenesis inhibitory activity to be used in the present invention may be formulated as salts thereof or complexes prepared by known pharmaceutical preparation methods. For example, in order to increase their water-solubility, promote their absorption and increase their pharmacological activities, the fumagillol derivatives may be used as complexes with cyclodextrin compounds. The cyclodextrin compounds include cyclodextrins (e.g., α-cyclodextrin, β-cyclodextrin, γ-cyclodextrin, δ-cyclodextrin) and cyclodextrin derivatives. Examples of the cyclodextrin derivatives include cyclodextrins containing saccharide residues as substituent, such as glucosyl-α-, -β-, -γ-, and -δ-cyclodextrin, maltosyl-α-, -β-, -γ-, and -δ-cyclodextrin, maltotriosyl-α-, -β-, -γ-, and -δ-cyclodextrin, dimaltosyl-α-, -β-, -γ-, and -δ-cyclodextrin.

Preferred examples of the cyclodextrin compounds include maltosyl-α-, -β-, -γ-, and -δ-cyclodextrin, glucosyl-α-, -β-, -γ-, and -δ-cyclodextrin. In particular, maltosyl-β-cyclodextrin is preferred.

The above complexes of fumagillol derivatives or salts and cyclodextrin compounds can be prepared, for example, by the method described in JP-A 4-297,469 (EP 461427)(e.g., the method wherein a fumagillol derivative or a salt thereof and a cyclodextrin compound are dissolved in water, and the solution is stirred at room temperature (-10°C to 35°C) to 80°C), and the obtained solution is filtered and then lyophilized to give powders ) or modifications thereof.

Examples of the platinum complex having cytocidal activity to be used in the present invention include platinum complexes having DNA-alkylating action. Specific examples thereof include cisplatin, carboplatin, nedaplatin, zeniplatin, enloplatin, lobaplatin, ormaplatin, loboplatin and sebriplatin. In particular, cisplatin is preferred.

The antineoplastic pharmaceutical composition for plural administrations to be used in the present invention is normally used orally or parenterally and comprises the above active components and a pharmaceutically acceptable carrier, excipient or diluent. For example, the composition may be in the form of a combination of aqueous solutions of the respective active components, a solid mixture prepared by mixing respective lyophilized active components, a combination of respective lyophilized aqueous solutions of active components, a combination of an aqueous solution of one active component and the other lyophilized components, a set (kit) wherein respective components are formulated into separate preparations .

The composition of the present invention can be administered as a single agent prepared by mixing the active components optionally together with a pharmaceutically acceptable carrier, excipient, diluent or the like according to known pharmaceutical preparation methods. Further, respective active components can be formulated into separate preparations by optionally using a pharmaceutically acceptable carrier, excipient or diluent, and can be administered as a single agent by mixing them with a diluent or the like just before use. Furthermore, as described above, separately prepared formulations can be administered simultaneously or separately at intervals to the same subject by the same or different administration routes. The above formulations can be assembled and used as a kit.

In any administration method, each of the fumagillol derivative or a salt thereof having angiogenesis inhibitory activity and the platinum complex having cytocidal activity is administered to a subject at least twice.

When the composition of the present invention is in the form of a solution, it can be prepared according to conventional methods by using solvents such as aqueous solvents (e.g., distilled water), water-soluble solvents (e.g., physiological saline, Ringer's solution) and oily solvents (e.g., sesame oil, corn oil, olive oil). At this time, there can optionally be used additives such as solution adjuvants (e.g., sodium salicylate, sodium acetate), buffering agents (e.g., sodium citrate, glycerin), isotonicity agents (e.g., glucose, invert sugar), stabilizing agent (e.g., human serum albumin, polyethylene glycol), preservatives (e.g., benzyl alcohol, phenol)and analgesics (e.g., benzalkonium chloride, procaine hydrochloride).

The amount of TNP and the platinum complex in the preparation varies with the formulation. For example, the concentration of the platinum complex in a solution is preferably 0.05 to 5 mg/ml, more preferably 0.1 to 1 mg/ml. The concentration of TNP in a solution is preferably 0.1 to 100 mg/ml. In particular, the concentration of TNP is preferably 5 to 50 mg/ml for subcutaneous or intravenous administration and 0.1 to 4 mg/ml for instillation.

The amount of TNP to be used varies with a particular combination with the platinum complex having cytocidal activity. For example, it is 0.5 to 1000 times (by weight), preferably 1 to 1000 times (by weight), that of the platinum complex.

Other examples of the composition for oral administration include tablets, pills, granules, powders, capsules, syrups, emulsions and suspensions. Such compositions can be prepared by per se known method. As a carrier or excipient, there can be used lactose, starch, sucrose and magnesium stearate.

For parenteral administration, for example, injections, suppositories and external preparations can be employed. The injections can be in the form of intravenous injections, subcutaneous injections, intramuscular injections and drip injections. The injections are usually provided as preparations filled into appropriate ampules.

Examples of suppositories are rectal suppositories and vaginal suppositories. Examples of external preparations are ointments including creams, pernasal preparations and percutaneous preparations.

To prepare external preparations, for example, the composition of the present invention can be molded into solid, semisolid or liquid external preparations. For the above solid preparations, the composition of the present invention is molded into powder composition as it is or after mixing it with added vehicles (e.g., glucose, mannitol, starch, microcrystalline cellulose) and thickening agents (e.g., natural gums, cellulose derivatives, acrylic acid polymers).

For the above liquid preparations, oily or aqueous suspensions are prepared according to almost the same manner as that in the above injectable preparations. As the above semisolid preparations, aqueous or oily gels or ointments are preferred. Each of these preparations may contain pH adjustors (e.g., carbonic acid, phosphoric acid, citric acid, hydrochloric acid, sodium hydroxide) and antiseptics (e.g., p-hydroxybenzoic acid esters, chlorobutanol, benzalkonium chloride ).

To prepare suppositories, for example, the composition of the present invention can be molded by per se known methods into oily or aqueous solid, semisolid or liquid suppositories.

The antineoplastic pharmaceutical composition of the present invention is useful for treating tumors in tumor-bearing mammals (e.g., mice, rats, rabbits, cats, dogs, cattle, horses, goats, monkeys, humans), and is significantly effective for apothanasia (i.e. the prolongation of life) and against cancer metastases in these tumor-bearing animals. Examples of tumors to be treated include various malignant and benign tumors such as malignant melanoma, malignant lymphoma, digestive apparatus (e.g., stomach, bowel) cancer, lung cancer, pancreatic cancer, esophagus cancer, breast carcinoma, hepatic cancer, ovarian cancer, uterine cancer, prostatic cancer, brain tumor, Kaposi's sarcoma, hemangioma, osteosarcoma and myosarcoma. The present invention is especially effective for the treatment of breast carcinoma, hepatic cancer, prostatic cancer, digestive apparatus cancer, lung cancer, brain tumor and Kaposi's sarcoma.

The composition of the present invention has low toxicity and can be administered orally or parenterally to mammals including humans. As described above, pharmacologically and pharmaceutically acceptable additives (e.g., carriers, diluents, excipients, binders, disintegrants, colorants, stabilizing agents ) can optionally be added or used for formulation. The dose of the composition of the present invention varies with the dosage form, mode of administration, etc. For example, when administered to mice as injections, it is preferably 0.1 to 200 mg/kg per day of TNP and 0.01 to 100 mg/kg per day of the platinum complex having cytocidal activity. When administered to mammals other than mice, it is preferably 0.001 to 100 mg/kg per day of TNP and 0.001 to 100 mg/kg per day of the platinum complex. More preferably, it is 1 to 100 mg/kg per day of TNP and 0.01 to 50 mg/kg per day of the platinum complex.

In the antineoplastic pharmaceutical composition of the present invention, separate preparations of TNP and the platinum complex having cytocidal activity con be administered to the same subject simultaneously or at an interval in any order of administrations depending upon, for example, conditions of the patients.

According to the present invention, excellent antineoplastic activity can be obtained by plural administrations of a combination of a fumagillol derivative having angiogenesis inhibitory activity or a salt thereof and a platinum complex having cytocidal activity. Further, the combination reduces side effects.

The following reference examples, examples and experiments further illustrate the present invention in detail.

Reference Example 1

Preparation of a complex (inclusion compound) of 6-O-(N-chloroacetylcarbamoyl)fumagillol and maltosyl-β-cyclodextrin

Maltosyl-β-cyclodextrin (719 g) was dissolved in water, and then 6-O-(N-chloroacetylcarbamoyl)fumagillol (hereinafter sometimes referred to as Compound A)(99 g) was added. The mixture was stirred at 25°C for 3 hours using a stirrer to give a solution (4950 ml). After checking that most of Compound A was dissolved, the mixture was filtered through a filter having a pore size of 0.22 μm. The resulting drug solution (5 ml/vial) was charged into vials and lyophilized to give an inclusion compound.

Example 1

An inclusion compound (30 mg, which contains 3.6 mg of Compound A) obtained according to the method of Reference Example 1 and the injection Randa (trade name, manufactured by Nippon Kayaku Co., LTD., Japan) (20 ml) containing cisplatin (0.5 mg/ml) were mixed, charged into vials and lyophilized to give a preparation containing Compound A and cisplatin.

Example 2

Five vials of the injection Randa (trade name, manufactured by Nippon Kayaku, Co., Ltd., Japan) (50 ml/vial) containing cisplatin (0.5 mg/ml) and 5 vials containing the inclusion compound obtained in Reference Example 1 were made a set.

Example 3

An inclusion compound (247 mg which comprises 30 mg of Compound A) obtained according to the method of Reference Example 1 and the injection Randa (trade name, manufactured by Nippon Kayaku Co., LTD., Japan) (20 ml) containing cisplatin (0.5 mg/ml) were mixed, charged into vials and lyophilized to give a preparation containing Compound A and cisplatin.

Experiment 1

Tumor growth inhibitory activity of 6-O-(N-chloroacetylcarbamoyl)fumagillol (Compound A) and cisplatin in their single and concomitant plural administrations to subcutaneously transplanted tumors

Hormone-independent human prostatic cancer PC-3 strain cells (hereinafter abbreviated as PC-3 cells) subcultured in vitro using a petri dish for tissue culture were removed from the dish wall by using 0.25% trypsin. The cells thus obtained were rinsed with Ham F-12K medium containing 10% serum and suspended in PBS (phosphate-buffered saline) so that the concentration of the cells became 3 x $10^7$/ml, and 100 μl of the resulting suspension was transplanted to the central part of the right side of the abdomens of Balb/c nu/nu mice (9 weeks old, male) with an injection syringe (25G needle). The tumor size was determined on the 17th day. The mice were divided into groups so that the mean tumor size of each groups was 96 to 97 $mm^3$. Then administration of the drug prepared by the following method was started. The tumor size was determined with a caliper in terms of the major axis (a) and minor axis (b), and calculated from the following formula: $0.5 \times a \times b^2$.

A 5% glucose solution was added to an inclusion compound of Compound A and maltosyl-β-cyclodextrin obtained in Reference Example 1 to adjust a dosage volume to 10 μl/g body weight of each mouse, and the resulting solution was administered to dorsal subcutaneous parts of mice.

The injection Randa (trade name, manufactured by Nippon Kayaku K.K., Japan) was used as cisplatin, dissolved in saline, adjusted to a dosage volume of 10 μl/g body weight of each mouse, and intraperitoneally administered to mice.

In the case of administration of Compound A alone and cisplatin alone, the first administration was carried out on the 17th day after the transplantation, and thereafter administrations were carried out at one-week intervals. In the case of concomitant administration of Compound A and cisplatin, cisplatin was administered on the 17th day after the transplantation, and Compound A was administered on the 21th day after the transplantation. Thereafter, each drug was administered at one-week intervals.

Tumor growth inhibitory activity was evaluated on the 49th day after the transplantation as follows. Each tumor size was determined on the 49th day, the tumor size on the day of the first administration was subtracted from the size

on the 49th day, and the ratio (T/C%) of the tumor size of the drug administration group (T) to that of the control group (C) was calculated. The results are in Table 1. The dose of the drug is indicated in terms of the weight of the drug (mg/kg body weight) per administration.

Table 1

| Dose (mg/kg) | | Number of animals | Tumor size gain ($mm^3$) mean±SD | Tumor size ratio (T/C %) |
|---|---|---|---|---|
| Compound A | Cisplatin | | | |
| 0 | 0 | 5 | 1219±376 | |
| 0 | 4 | 5 | 230±182 | 19 |
| 100 | 0 | 4 | 591±242 | 49 |
| 100 | 4 | 5 | 50± 36 | 4 |

Excellent tumor growth inhibitory activity of a combination of Compound A and cisplatin was observed.

Experiment 2

Tumor growth inhibitory activity of Compound A and cisplatin in their single and concomitant plural administrations to subcutaneously transplanted tumors

Mice bearing PC-3 cancer cells were prepared according to the same manner as that in Experiment 1. The tumor size was determined on the 17th day after the transplantation. Mice were divided into groups so that the average tumor size of each group was 108 to 109 $mm^3$. Then drug administration was started. Preparation of the drug, determination of the tumor size, evaluation of tumor growth inhibitory activity, etc., were carried out according to the methods of Experiment 1.

In the case of administration of Compound A alone and cisplatin alone, the first administration was carried out on the 17th day after the transplantation, and thereafter administrations were carried out at one-week intervals.

The following three combinations of the drugs were administered. Cisplatin and Compound A were administered on the 17th day and 21th day after the transplantation, respectively, and thereafter each drug was administered at one-week intervals. These administrations are hereinafter referred to as Combination 1. Compound A and cisplatin were administered on the 17th day and 21th day after the transplantation, respectively, and thereafter each drug was administered at one-week intervals. These administrations are hereinafter referred to as Combination 2. Compound A and cisplatin were administered simultaneously on the 17th day after the transplantation, and thereafter each drug was administered at on-week intervals. These administrations are hereinafter referred to as Combination 3.

On the 42th day after the tumor transplantation, tumor growth inhibitory activity was evaluated according to the same manner as in Experiment 1. The results are in Table 2.

Table 2

| Dose (mg/kg) | | Number of animals | Tumor size gain ($mm^3$) means±SD | Tumor size ratio (T/C %) |
|---|---|---|---|---|
| Compound A | Cisplatin | | | |
| 0 | 0 | 5 | 1327±286 | |
| 0 | 5 | 5 | 345±163 | 26 |
| 100 | 0 | 5 | 400±126 | 30 |
| 100 | 5 (Combination 1) | 5 | 205±103 | 15 |
| 100 | 5 (Combination 2) | 5 | 130± 43 | 10 |
| 100 | 5 (Combination 3) | 5 | 105±126 | 8 |

In any dosage schedules of Combinations 1, 2 and 3, excellent tumor growth inhibitory activity of a combination of Compound A and cisplatin was observed.

## Claims

1. An antineoplastic pharmaceutical composition for plural administrations which consists of a fumagillol derivative having angiogenesis inhibitory activity or a salt thereof in combination with a platinum complex having cytocidal activity, and if necessary has a pharmaceutically acceptable carrier or excipient.

2. A pharmaceutical composition according to claim 1, wherein the fumagillol derivative is a compound of the formula (I):

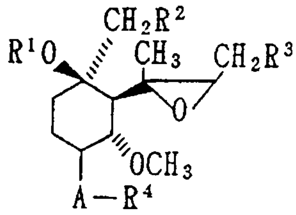

wherein $R^1$ is hydrogen; $R^2$ is halogen, $N(O)_mR^5R^6$, $N^+R^5R^6R^7 \cdot X^-$, $S(O)_nR^5$ or $S^+R^5R^6 \cdot X^-$, in which $R^5$, $R^6$ and $R^7$ represent independently an optionally substituted hydrocarbon group or an optionally substituted heterocyclic group; $X^-$ represents a counter anion; m represents 0 or 1; n represents an integer of 0 to 2; or $R^5$ and $R^6$ together with the adjacent nitrogen or sulfur atom may form an optionally substituted nitrogen- or sulfur-containing heterocyclic group which may form a condensed ring; or $R^1$ and $R^2$ are combined to represent a chemical bond; $R^3$ is an optionally substituted 2-methyl-1-propenyl group or an optionally substituted isobutyl group; A is oxygen or $NR^8$, in which $R^8$ represents hydrogen, an optionally substituted lower alkyl group or an optionally substituted aryl group; and $R^4$ is hydrogen, an optionally substituted hydrocarbon group or an optionally substituted acyl group.

3. A pharmaceutical composition according to claim 2, wherein $R^1$ and $R^2$ are combined to represent a chemical bond.

4. A pharmaceutical composition according to claim 2, wherein the optionally substituted hydrocarbon group represented by $R^5$, $R^6$ or $R^7$ is a straight-chain or branched $C_{1-6}$ alkyl, $C_{2-6}$ alkenyl or $C_{2-6}$ alkynyl group, a $C_{3-6}$ cycloalkyl group, a $C_{3-6}$ cycloalkenyl group, a $C_{7-13}$ aralkyl group or a $C_{6-10}$ aryl group, each of which may optionally be substituted.

5. A pharmaceutical composition according to claim 2, wherein A is oxygen.

6. A pharmaceutical composition according to claim 2, wherein $R^3$ is 2-methyl-1-propenyl.

7. A pharmaceutical composition according to claim 2, wherein $R^4$ is an N-substituted carbamoyl group.

8. A pharmaceutical composition according to claim 1, wherein the fumagillol derivative is 6-O-(N-chloroacetylcarbamoyl)fumagillol.

9. A pharmaceutical composition according to claim 1, wherein the fumagillol derivative is 6-O-(N-methylcarbamoyl)fumagillol.

10. A pharmaceutical composition according to claim 1, wherein the fumagillol derivative or a salt thereof is in the form of a complex with a cyclodextrin compound.

11. A pharmaceutical composition according to claim 10, wherein the cyclodextrin compound is maltosyl-β-cyclodextrin.

12. A pharmaceutical composition according to claim 1, wherein the platinum complex shows DNA-alkylating action.

13. A pharmaceutical composition according to claim 1, wherein the platinum complex is selected from the group consisting of cisplatin, carboplatin, nedaplatin, zeniplatin, enloplatin, lobaplatin, ormaplatin, loboplatin and sebriplatin.

**14.** A pharmaceutical composition according to claim 13, wherein the platinum complex is cisplatin.

**15.** A pharmaceutical composition according to claim 1, wherein the composition is for treating malignant melanoma, malignant lymphoma, digestive apparatus cancer, lung cancer, pancreatic cancer, esophagus cancer, breast carcinoma, hepatic cancer, ovarian cancer, uterine cancer, prostatic cancer, brain tumor, Kaposi's sarcoma, hemangioma, osteosarcoma or myosarcoma.

**16.** A pharmaceutical composition according to claim 15, wherein the composition is for treating breast carcinoma, hepatic cancer, prostatic cancer, digestive apparatus cancer, lung cancer, brain tumor or Kaposi's sarcoma.

**17.** A pharmaceutical composition according to claim 1, wherein the composition is for treating tumor in a mammal and is suitable for being administered to such a mammal at least twice.

**18.** A kit of antineoplastic preparations for plural administration which comprises (1) a preparation comprising a fumagillol derivative having angiogenesis inhibitory activity or a salt thereof and a pharmaceutically acceptable carrier or excipient, and (2) a preparation comprising a platinum complex having cytocidal activity and a pharmaceutical carrier or excipient.

**19.** Use of a composition consisting of a fumagillol derivative having angiogenesis inhibitory activity or a salt thereof in combination with a platinum complex having cytocidal activity for preparing a medicament for treating a tumor in a mammal.

**20.** The use of claim 19 wherein the tumor is malignant melanoma, malignant lymphoma, digestive apparatus cancer, lung cancer, pancreatic cancer, esophagus cancer, breast carcinoma, hepatic cancer, ovarian cancer, uterine cancer, prostatic cancer, brain tumor, Kaposi's sarcoma, hemangioma, osteosarcoma or myosarcoma.

**21.** The use of claim 20 wherein the tumor is breast carcinoma, hepatic cancer, prostatic cancer, digestive apparatus cancer, lung cancer, brain tumor or Kaposi's sarcoma.

**22.** The use according to any of claims 19-21 wherein the medicament further comprises a pharmaceutically acceptable carrier or excipient.

**23.** The use according to any of claims 19-22 wherein the medicament comprises (1) a preparation comprising an antineoplastically effective amount of a fumagillol derivative having angiogenesis inhibitory activity or a salt thereof and a pharmaceutically acceptable carrier or excipient in combination with (2) a preparation comprising an antineoplastically effective amount of platinum complex having cytocidal activity and a pharmaceutically eptable carrier or excipient.

**24.** The use according to any of claims 19-23 wherein the fumagillol derivative is a compound of the formula (I):

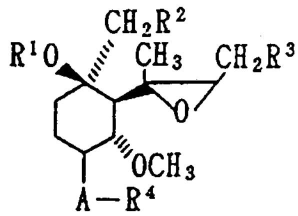

wherein $R^1$ is hydrogen; $R^2$ is halogen, $N(O)_mR^5R^6$, $N^+R^5R^6R^7 \cdot X^-$, $S(O)_nR^5$ or $S^+R^5R^6 \cdot X^-$, in which $R^5$, $R^6$ and $R^7$ represent independently an optionally substituted hydrocarbon group or an optionally substituted heterocyclic group; $X^-$ represents a counter anion; m represents 0 or 1; n represents an integer of 0 to 2; or $R^5$ and $R^6$ together with the adjacent nitrogen or sulfur atom may form an optionally substituted nitrogen- or sulfur-containing heterocyclic group which may form a condensed ring; or $R^1$ and $R^2$ are combined to represent a chemical bond; $R^3$ is an optionally substituted 2-methyl-1-propenyl group or an optionally substituted isobutyl group; A is oxygen or $NR^8$, In which $R^8$ represents hydrogen, an optionally substituted lower alkyl group or an optionally substituted aryl group; and $R^4$ is hydrogen, an optionally substituted hydrocarbon group or an optionally substituted acyl group.

**25.** Use of a fumagillol derivative having angiogenesis inhibitory activity in combination with a platinum complex having cytocidal activity for formulation of an antineoplastic pharmaceutical composition which consists of a fumagillol derivative having angiogenesis inhibitory activity or a salt thereof in combination with a platinum complex having cytocidal activity, and if necessary has a pharmaceutically acceptable carrier or excipient for plural administration.

## Patentansprüche

**1.** Antineoplastische pharmazeutische Zusammensetzung für mehrfache Verabreichung, die aus einem Fumagillol-Derivat mit Angiogenese-hemmender Wirkung oder einem Salz davon in Kombination mit einem Platinkomplex mit cytozider Wirkung besteht und, falls notwendig, einen pharmazeutisch annehmbaren Träger oder Arzneimittelhilfsstoff aufweist.

**2.** Pharmazeutische Zusammensetzung gemäß Anspruch 1, wobei das Fumagillol-Derivat eine Verbindung der Formel (I)

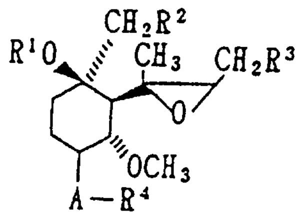

ist, wobei $R^1$ Wasserstoff ist, $R^2$ Halogen, $N(O)_mR^5R^6$, $N^+R^5R^6R^7 \cdot X^-$, $S(O)_nR^5$ oder $S^+R^5R^6 \cdot X^-$ ist, wobei $R^5$, $R^6$ und $R^7$ unabhängig eine gegebenenfalls substituierte Kohlenwasserstoffgruppe oder eine gegebenenfalls substituierte heterocyclische Gruppe sind, $X^-$ ein Gegenanion ist, m 0 oder 1 ist, n eine ganze Zahl von 0 bis 2 ist oder $R^5$ und $R^6$ zusammen mit dem benachbarten Stickstoff- oder Schwefelatom eine gegebenenfalls substituierte stickstoff- oder schwefelhaltige heterocyclische Gruppe bilden können, die einen kondensierten Ring bilden kann, oder $R^1$ und $R^2$ miteinander kombiniert sind, so daß sie eine chemische Bindung darstellen, $R^3$ eine gegebenenfalls substituierte 2-Methyl-1-propenylgruppe oder eine gegebenenfalls substituierte Isobutylgruppe ist, A Sauerstoff oder $NR^8$ ist, wobei $R^8$ Wasserstoff, eine gegebenenfalls substituierte Niederalkylgruppe oder eine gegebenenfalls substituierte Arylgruppe ist, und $R^4$ Wasserstoff, eine gegebenenfalls substituierte Kohlenwasserstoffgruppe oder eine gegebenenfalls substituierte Acylgruppe ist.

**3.** Pharmazeutische Zusammensetzung gemäß Anspruch 2, wobei $R^1$ und $R^2$ miteinander kombiniert sind, so daß sie eine chemische Bindung darstellen.

**4.** Pharmazeutische Zusammensetzung gemäß Anspruch 2, wobei die durch $R^5$, $R^6$ oder $R^7$ dargestellte gegebenenfalls substituierte Kohlenwasserstoffgruppe eine geradkettige oder verzweigte $C_{1-6}$-Alkyl-, $C_{2-6}$-Alkenyl- oder $C_{2-6}$-Alkinylgruppe, eine $C_{3-6}$-Cycloalkylgruppe, eine $C_{3-6}$-Cycloalkenylgruppe, eine $C_{7-13}$-Aralkylgruppe oder eine $C_{6-10}$-Arylgruppe ist, von denen jede gegebenenfalls substituiert sein kann.

**5.** Pharmazeutische Zusammensetzung gemäß Anspruch 2, wobei A Sauerstoff ist.

**6.** Pharmazeutische Zusammensetzung gemäß Anspruch 2, wobei $R^3$ 2-Methyl-1-propenyl ist.

**7.** Pharmazeutische Zusammensetzung gemäß Anspruch 2, wobei $R^4$ eine N-substituierte Carbamoylgruppe ist.

**8.** Pharmazeutische Zusammensetzung gemäß Anspruch 1, wobei es sich bei dem Fumagillol-Derivat um 6-O-(N-Chloracetylcarbamoyl)fumagillol handelt.

**9.** Pharmazeutische Zusammensetzung gemäß Anspruch 1, wobei es sich bei dem Fumagillol-Derivat um 6-O-(N-Methylcarbamoyl)fumagillol handelt.

**10.** Pharmazeutische Zusammensetzung gemäß Anspruch 1, wobei das Fumagillol-Derivat oder das Salz davon in Form eines Komplexes mit einer Cyclodextrinverbindung vorliegt.

**11.** Pharmazeutische Zusammensetzung gemäß Anspruch 10, wobei es sich bei der Cyclodextrinverbindung um Maltosyl-β-cyclodextrin handelt.

**12.** Pharmazeutische Zusammensetzung gemäß Anspruch 1, wobei der Platinkomplex eine DNA-alkylierende Wirkung zeigt.

**13.** Pharmazeutische Zusammensetzung gemäß Anspruch 1, wobei der Platinkomplex aus der Gruppe ausgewählt ist, die aus Cisplatin, Carboplatin, Nedaplatin, Zeniplatin, Enloplatin, Lobaplatin, Ormaplatin, Loboplatin und Sebriplatin besteht.

**14.** Pharmazeutische Zusammensetzung gemäß Anspruch 13, wobei es sich bei dem Platinkomplex um Cisplatin handelt.

**15.** Pharmazeutische Zusammensetzung gemäß Anspruch 1, wobei die Zusammensetzung zur Behandlung von malignen Melanomen, malignen Lymphomen, Krebs des Verdauungsapparats, Lungenkrebs, Bauchspeicheldrüsenkrebs, Speiseröhrenkrebs, Brustkarzinom, Leberkrebs, Eierstockkrebs, Gebärmutterkrebs, Prostatakrebs, Hirntumor, Kaposi-Sarkom, Hämangiom, Osteosarkom oder Myosarkom bestimmt ist.

**16.** Pharmazeutische Zusammensetzung gemäß Anspruch 15, wobei die Zusammensetzung zur Behandlung von Brustkarzinom, Leberkrebs, Prostatakrebs, Krebs des Verdauungsapparats, Lungenkrebs, Hirntumor oder Kaposi-Sarkom bestimmt ist.

**17.** Pharmazeutische Zusammensetzung gemäß Anspruch 1, wobei die Zusammensetzung zur Behandlung von Tumoren bei einem Säuger bestimmt und geeignet ist, einem solchen Säuger wenigstens zweimal verabreicht zu werden.

**18.** Kit mit antineoplastischen Präparaten für mehrfache Verabreichung, umfassend (1) ein Präparat, das ein Fumagillol-Derivat mit Angiogenese-hemmender Wirkung oder ein Salz davon sowie einen pharmazeutisch annehmbaren Träger oder Arzneimittelhilfsstoff umfaßt, sowie (2) ein Präparat, das einen Platinkomplex mit cytozider Wirkung sowie einen pharmazeutisch annehmbaren Träger oder Arzneimittelhilfsstoff umfaßt.

**19.** Verwendung einer Zusammensetzung, die aus einem Fumagillol-Derivat mit Angiogenese-hemmender Wirkung oder einem Salz davon in Kombination mit einem Platinkomplex mit cytozider Wirkung besteht, zur Herstellung eines Medikaments zur Behandlung eines Tumors bei einem Säuger.

**20.** Verwendung gemäß Anspruch 19, wobei es sich bei dem Tumor um malignes Melanom, malignes Lymphom, Krebs des Verdauungsapparats, Lungenkrebs, Bauchspeicheldrüsenkrebs, Speiseröhrenkrebs, Brustkarzinom, Leberkrebs, Eierstockkrebs, Gebärmutterkrebs, Prostatakrebs, Hirntumor, Kaposi-Sarkom, Hämangiom, Osteosarkom oder Myosarkom handelt.

**21.** Verwendung gemäß Anspruch 20, wobei es sich bei dem Tumor um Brustkarzinom, Leberkrebs, Prostatakrebs, Krebs des Verdauungsapparats, Lungenkrebs, Hirntumor oder Kaposi-Sarkom handelt.

**22.** Verwendung gemäß einem der Ansprüche 19-21, wobei das Medikament weiterhin einen pharmazeutisch annehmbaren Träger oder Arzneimittelhilfsstoff umfaßt.

**23.** Verwendung gemäß einem der Ansprüche 19-22, wobei das Medikament (1) ein Präparat, das eine antineoplastisch wirksame Menge eines Fumagillol-Derivats mit Angiogenesehemmender Wirkung oder ein Salz davon sowie einen pharmazeutisch annehmbaren Träger oder Arzneimittelhilfsstoff umfaßt, in Kombination mit (2) einem Präparat, das eine antineoplastisch wirksame Menge eines Platinkomplexes mit cytozider Wirkung sowie einen pharmazeutisch annehmbaren Träger oder Arzneimittelhilfsstoff umfaßt, umfaßt.

**24.** Verwendung gemäß einem der Ansprüche 19-23, wobei das Fumagillol-Derivat eine Verbindung der Formel (I)

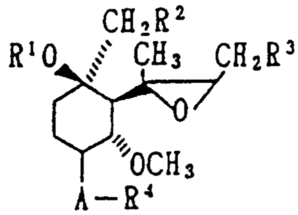

ist, wobei $R^1$ Wasserstoff ist, $R^2$ Halogen, $N(O)_mR^5R^6$, $N^+R^5R^6R^7 \cdot X^-$, $S(O)_nR^5$ oder $S^+R^5R^6 \cdot X^-$ ist, wobei $R^5$, $R^6$ und $R^7$ unabhängig eine gegebenenfalls substituierte Kohlenwasserstoffgruppe oder eine gegebenenfalls substituierte heterocyclische Gruppe sind, $X^-$ ein Gegenanion ist, m 0 oder 1 ist, n eine ganze Zahl von 0 bis 2 ist oder $R^5$ und $R^6$ zusammen mit dem benachbarten Stickstoff- oder Schwefelatom eine gegebenenfalls substituierte stickstoff- oder schwefelhaltige heterocyclische Gruppe bilden können, die einen kondensierten Ring bilden kann, oder $R^1$ und $R^2$ miteinander kombiniert sind, so daß sie eine chemische Bindung darstellen, $R^3$ eine gegebenenfalls substituierte 2-Methyl-1-propenylgruppe oder eine gegebenenfalls substituierte Isobutylgruppe ist, A Sauerstoff oder $NR^8$ ist, wobei $R^8$ Wasserstoff, eine gegebenenfalls substituierte Niederalkylgruppe oder eine gegebenenfalls substituierte Arylgruppe ist, und $R^4$ Wasserstoff, eine gegebenenfalls substituierte Kohlenwasserstoffgruppe oder eine gegebenenfalls substituierte Acylgruppe ist.

25. Verwendung eines Fumagillol-Derivats mit Angiogenesehemmender Wirkung in Kombination mit einem Platinkomplex mit cytozider Wirkung zur Zubereitung einer antineoplastischen pharmazeutischen Zusammensetzung, die aus einem Fumagillol-Derivat mit Angiogenese-hemmender Wirkung oder einem Salz davon in Kombination mit einem Platinkomplex mit cytozider Wirkung besteht und, falls notwendig, einen pharmazeutisch annehmbaren Träger oder Arzneimittelhilfsstoff aufweist, für mehrfache Verabreichung.

**Revendications**

1. Composition pharmaceutique antinéoplasique pour des administrations répétées comprenant un dérivé de fumagillol ayant une activité inhibitrice de l'angiogenèse ou un sel d'un tel composé en combinaison avec un complexe de platine ayant une activité cytotoxique et, si nécessaire, un véhicule ou excipient pharmaceutiquement acceptable.

2. Composition pharmaceutique selon la revendication 1, dans laquelle le dérivé de fumagillol est un composé de formule (I)

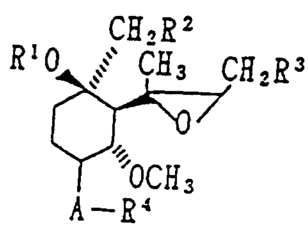

dans laquelle $R^1$ représente un atome d'hydrogène, $R^2$ représente un atome d'halogène ou un groupe $N(O)_mR^5R^6$, $N^+R^5R^6R^7X^-$, $S(O)_nR^5$ ou $S^+R^5R^6X^-$, où $R^5$, $R^6$ et $R^7$ représentent, indépendamment les uns des autres, un groupe hydrocarboné éventuellement substitué ou un groupe hétérocyclique éventuellement substitué, $X^-$ représente un contre-anion, m vaut 0 ou 1, n représente un nombre entier compris entre 0 et 2, ou $R^5$ et $R^6$ représentent en conjonction avec l'atome d'azote ou de soufre adjacent un groupe hétérocyclique azoté ou soufré éventuellement substitué et qui peut former un cycle condensé ; ou $R^1$ et $R^2$ représentent conjointement une liaison chimique ; $R^3$ représente un groupe 2-méthyl-1-propényle éventuellement substitué ou un groupe isobutyle éventuellement substitué ; A représente un atome d'oxygène ou un groupe $NR^8$ où $R^8$ représente un atome d'hydrogène, un groupe alkyle inférieur éventuellement substitué ou un groupe aryle éventuellement substitué, et $R^4$ est un atome d'hydrogène, un groupe hydrocarboné éventuellement substitué ou un groupe acyle éventuellement substitué.

3. Composition pharmaceutique selon la revendication 2, dans laquelle $R^1$ et $R^2$ représentent conjointement une

liaison chimique.

4. Composition pharmaceutique selon la revendication 2, dans laquelle le groupe hydrocarboné éventuellement substitué représenté par $R^5$, $R^6$ ou $R^7$ est un groupe alkyle à chaîne linéaire ou ramifiée en $C_{1-6}$, un groupe alcényle en $C_{2-6}$ ou alcynyle en $C_{2-6}$, un groupe cycloalkyle en $C_{3-6}$, un groupe cycloalcényle en $C_{3-6}$, un groupe aralkyle en $C_{7-13}$ ou un groupe aryle en $C_{6-10}$, chacun de ces groupes pouvant être substitué.

5. Composition pharmaceutique selon la revendication 2 où A représente un atome d'oxygène.

6. Composition pharmaceutique selon la revendication 2, où $R^3$ représente un groupe 2-méthyl-1-propényle.

7. Composition pharmaceutique selon la revendication 2, où $R^4$ représente un groupe carbamoyle N-substitué.

8. Composition pharmaceutique selon la revendication 1, dans laquelle le dérivé de fumagillol est le 6-O-(N-chloroacétylcarbamoyl)fumagillol.

9. Composition selon la revendication 1, dans laquelle le dérivé de fumagillol est le 6-O-(N-méthylcarbamoyl)fumagillol.

10. Composition pharmaceutique selon la revendication 1, dans laquelle le dérivé fumagillol ou un sel d'un tel composé est sous la forme d'un complexe avec une cyclodextrine.

11. Composition pharmaceutique selon la revendication 10, dans laquelle la cyclodextrine est la maltosyl-β-cyclodextrine.

12. Composition pharmaceutique selon la revendication 1, dans laquelle le complexe de platine présente une activité d'alkylation d'ADN.

13. Composition pharmaceutique selon la revendication 1 dans laquelle le complexe de platine est choisi dans le groupe formé par le cisplatine, le carboplatine, le nédaplatine, le zéniplatine, l'enloplatine, le lobaplatine, l'ormaplatine, le loboplatine et le sébriplatine.

14. Composition pharmaceutique selon la revendication 13, dans laquelle le complexe de platine est le cisplatine.

15. Composition pharmaceutique selon la revendication 1, laquelle composition est destinée au traitement de mélanomes malins, de lymphomes malins, du cancer de l'appareil digestif, du cancer des poumons, du cancer du pancréas, du cancer de l'oesophage, du carcinome de poitrine, du cancer du foie, du cancer des ovaires, du cancer de l'utérus, du cancer de la prostate, des tumeurs du cerveau, du sarcome de Kaposi, de d'hémangiomes, d'ostéosarcomes ou de myosarcomes.

16. Composition pharmaceutique selon la revendication 15, laquelle composition est destinée au traitement du carcinome de poitrine, du cancer du foie, du cancer de la prostate, du cancer de l'appareil digestif, du cancer des poumons, des tumeurs du cerveau ou du sarcome de Kaposi.

17. Composition pharmaceutique selon la revendication 1, dans laquelle la composition est destinée au traitement de tumeurs chez des mammifères et est appropriée pour être administré au moins deux fois auxdits mammifères.

18. Kit de préparations antinéoplasiques destinées à une administration répétée, lequel kit comprend

(1) une préparation comprenant un dérivé de fumagillol ayant une activité inhibitrice de l'angiogenèse ou un sel d'un tel composé, et un véhicule ou excipient pharmaceutiquement acceptable, et
(2) une préparation comprenant un complexe de platine ayant une activité cytotoxique et un véhicule ou excipient pharmaceutiquement acceptable.

19. Utilisation d'une composition constituée d'un dérivé de fumagillol ayant une activité inhibitrice de l'angiogenèse ou d'un sel d'un tel composé en combinaison avec un complexe de platine ayant une activité cytotoxique, pour la préparation d'un médicament destiné au traitement du cancer chez des mammifères.

**20.** Utilisation selon la revendication 19, dans laquelle la tumeur est un mélanome malin, un lymphome malin, un cancer de l'appareil digestif, un cancer des poumons, un cancer du pancréas, un cancer de l'oesophage, un carcinome de poitrine, un cancer du foie, un cancer des ovaires, un cancer de l'utérus, un cancer de la prostate, une tumeur du cerveau, le sarcome de Kaposi, un hémangiome, un ostéosarcome ou un myosarcome.

**21.** Utilisation selon la revendication 20 dans laquelle la tumeur est un carcinome de poitrine, un cancer du foie, un cancer de la prostate, un cancer de l'appareil digestif, un cancer des poumons, une tumeur du cerveau ou le sarcome de Kaposi.

**22.** Utilisation selon l'une quelconque des revendications 19 - 21, où le médicament comprend en outre un véhicule ou excipient pharmaceutiquement acceptable.

**23.** Utilisation selon l'une quelconque des revendications 19 - 22, dans laquelle le médicament comprend

(1) une préparation comprenant une quantité anti-néoplasique d'un dérivé de fumagillol ayant une activité inhibitrice de l'angiogenèse ou d'un sel d'un tel composé, et un véhicule ou excipient pharmaceutiquement acceptable, en combinaison avec
(2) une préparation comprenant une quantité antinéoplasique d'un complexe de platine ayant une activité cytotoxique et un véhicule ou excipient pharmaceutiquement acceptable.

**24.** Utilisation selon l'une quelconque des revendications 19 - 23, dans laquelle le dérivé de fumagillol est un composé de formule (I)

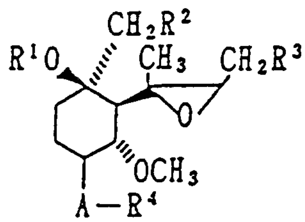

dans laquelle $R^1$ représente un atome d'hydrogène, $R^2$ représente un atome d'halogène ou un groupe $N(O)_mR^5R^6$, $N^+R^5R^6R^7X^-$, $S(O)_nR^5$ ou $S^+R^5R^6X^-$, où $R^5$, $R^6$ et $R^7$ représentent, indépendamment les uns des autres, un groupe hydrocarboné éventuellement substitué ou un groupe hétérocyclique éventuellement substitué, $X^-$ représente un contre-anion, m vaut 0 ou 1, n représente un nombre entier compris entre 0 et 2, ou $R^5$ et $R^6$ représentent en conjonction avec l'atome d'azote ou de soufre adjacent un groupe hétérocyclique azoté ou soufré éventuellement substitué et qui peut former un cycle condensé ; ou $R^1$ et $R^2$ représentent conjointement une liaison chimique ; $R^3$ représente un groupe 2-méthyl-1-propényle éventuellement substitué ou un groupe isobutyle éventuellement substitué ; A représente un atome d'oxygène ou un groupe $NR^8$ où $R^8$ représente un atome d'hydrogène, un groupe alkyle inférieur éventuellement substitué ou un groupe aryle éventuellement substitué, et $R^4$ est un atome d'hydrogène, un groupe hydrocarboné éventuellement substitué ou un groupe acyle éventuellement substitué.

**25.** Utilisation d'un dérivé de fumagillol ayant une activité inhibitrice de l'angiogenèse en combinaison avec un complexe de platine ayant une activité cytotoxique pour la formulation d'une composition pharmaceutique antinéoplasique constituée d'un dérivé de fumagillol ayant une activité inhibitrice de l'angiogenèse ou d'un sel d'un tel composé en combinaison avec un complexe de platine ayant une activité cytotoxique et, si nécessaire, d'un véhicule ou excipient pharmaceutiquement acceptable.